(19) Europäisches Patentamt
European Patent Office
Office européen des brevets

(11) Numéro de publication: **0 315 494 B1**

(12) # FASCICULE DE BREVET EUROPEEN

(45) Date de publication de fascicule du brevet: **08.12.93** (51) Int. Cl.[5]: **C07K 1/06**

(21) Numéro de dépôt: **88401050.5**

(22) Date de dépôt: **29.04.88**

(54) **Procédé de préparation de synthons peptidiques.**

(30) Priorité: **06.11.87 FR 8715398**

(43) Date de publication de la demande:
**10.05.89 Bulletin 89/19**

(45) Mention de la délivrance du brevet:
**08.12.93 Bulletin 93/49**

(84) Etats contractants désignés:
**AT BE CH DE ES FR GB GR IT LI LU NL SE**

(56) Documents cités:
**EP-A- 0 056 618**
**EP-A- 0 184 243**

**CHEMISCHE BERICHTE, vol. 94, no. 5, 1961, pages 1263-1267; L. BIRKOFER et al.: "Di-und Tripeptide aus silylierten Aminosäuren; der Trimethylsilylrest als Schutzgruppe für SH- und OH-Funktionen"**

**HOUBEN-WEYL: "Synthese von Peptiden", vol. 1, 1974, pages 28-31, Georg Thieme Verlag, Stuttgart, DE; E. WÜNSCH: "Grundlagen der Peptidsynthese"**

**CHEMICAL ABSTRACTS, vol. 85, no. 3, 19 juillet 1976, page 734, résumé no. 21797k, Columbus, Ohio, US; H.R. KRICHELDORF et al.: "Synthesis of polypeptides using diester triazolides of phosphorous acid", & ANGEW. CHEM. 1976, 88(10), 337-8**

(73) Titulaire: **RHONE-POULENC CHIMIE**
**25, quai Paul Doumer**
**F-92408 Courbevoie Cédex(FR)**

(72) Inventeur: **Jacquier, Robert**
**Rue Valéry Larbaud**
**F-34100 Montpellier(FR)**
Inventeur: **Verducci, Jean**
**12, rue Paul Gauguin**
**F-34670 Baillargues(FR)**
Inventeur: **Pevere, Virginie**
**Résidence Vivadi**
**Bat. C2**
**Avenue Paul Parguel**
**F-34090 Montpellier(FR)**

(74) Mandataire: **Le Pennec, Magali et al**
**RHONE-POULENC INTERSERVICES**
**Service Brevets Chimie**
**25, Ouai Paul Doumer**
**F-92408 Courbevoie Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3. 10/3.6/3.3. 1)

## Description

La présente invention concerne un procédé de préparation de synthons peptidiques. Elle concerne plus particulièrement une nouvelle méthode de synthèse peptidique non racémisante.

Il est connu d'après certains ouvrages spécialisés tels que par exemple (The Peptides, Vol. 1, Academic Press, 1979 ou Principles of peptides synthesis, Springer, 1984) de réaliser des synthèses peptidiques par condensation d'une chaîne peptidique dont la terminaison acide est activée (E) et la terminaison amine est protégée (P) avec une autre chaîne peptidique dont seule la terminaison acide est estérifiée en présence d'une base organique qui permet de neutraliser le groupe partant EOH qui est dans la majorité des cas acide selon les réactions suivantes :

activation

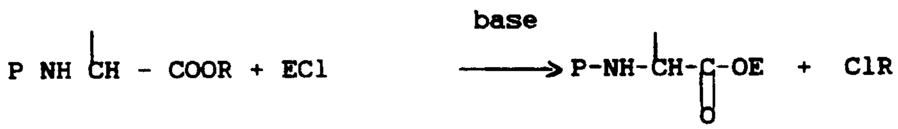


couplage

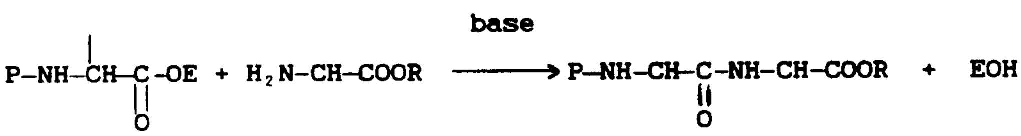


La base utilisée quelle qu'elle soit provoque une racémisation importante du motif peptidique soit au cours de l'étape d'activation soit au cours de l'étape de couplage.

Or, il est bien connu en synthèse peptidique que la plupart des peptides ne sont actifs que sous une seule forme diastéréoisomère. La racémisation provoque une perte d'activité des produits obtenus après la condensation, ce qui est très nuisible car les matières premières utilisées chiralement actives sont d'un prix souvent très élevé. Les peptides étant utilisés dans l'industrie pharmaceutique qui exige des normes analytiques sévères doivent être purifiés s'ils sont obtenus sous forme de mélange de diastéréoisomères lors de leur synthèse. Cette purification est très onéreuse.

L'industrie est donc à la recherche depuis longtemps de procédés chimiques concurrentiels des procédés d'extraction à partir des produits naturels qui permettent d'atteindre des peptides actifs d'une pureté chirale bien définie et à un coût pouvant concurrencer les procédés d'extraction.

Il est connu par exemple de préparer les peptides en inhibant au maximum la racémisation par l'utilisation d'agents activants tels que le dicyclohexylcarbodiimide (DCC) et d'additifs tels que :

le N-hydroxysuccinimide,
l'hydroxy-1 benzotriazole,
le N-hydroxy norbornène-5 dicarboximide-2,3.

Il est généralement connu de condenser sans trop de racémisation des aminoacides N-protégés par des groupes uréthannes par une technique de synthèse permettant d'additionner à un peptide les acides aminés un par un. Par contre, lorsque la fonction amine est substituée par un groupe acyle (Miyazawa, Yamada et Kuwata, Peptide Chemistry, 1982, 69) ou fait partie d'une chaîne peptidique, le taux de racémisation n'est plus négligeable et peut atteindre 25 %.

Le taux de racémisation varie avec l'acide aminé activé, le groupe protecteur, le réactif activant et les conditions de la réaction d'activation, et en particulier peut être total lorsque l'on utilise un groupe protecteur du type acyle.

La présente invention permet de résoudre les problèmes laissés dans l'art antérieur. Elle permet par une voie chimique à partir de matières premières peu onéreuses d'accéder à des synthons peptidiques présentant une pureté chirale égale ou supérieure à 99 % lors du couplage par exemple de la valine sur une autre valine, qui avec d'autres conditions expérimentales (activation du type : DCC et additif ou chlorure de pivaloyle et amine tertiaire ou BOP Cl et amine tertiaire) conduit à une racémisation non négligeable.

Elle a pour objet un procédé de préparation de synthons peptidiques optiquement actifs caractérisé en ce que :

- dans une première étape on prépare un dérivé O-silylé (I) d'un aminoacide ou d'un peptide dont la fonction azotée est protégée,
- dans une deuxième étape on active l'aminoacide ou le peptide O-silylé par un dérivé halogéné du phosphore (II),(X, $P^{III \text{ ou } V}$ avec X chlore ou brome),
- dans une troisième étape on condense l'aminoacide ou le peptide activé (III) sur un aminoacide ou un peptide dont la fonction amine a été N-sylilée.

La présente invention permet d'éviter donc toute intervention de base en particulier dans l'étape d'activation (B) et dans l'étape de couplage (C). Elle est particulièrement intéressante lorsque le peptide O-silylé contient plus d'un aminoacide.

Il est aussi connu d'après le brevet européen n° 184243 de préparer des dérivés silylés d'acides aminés ou de peptide à l'aide de trialkylcyanosilanes puis à coupler ces dérivés silylés avec des aminoacides ou des peptides activés. Lors de la silylation il y a libération d'acide cyanhydrique dont la toxicité est telle qu'elle a fait exclure ce procédé de tous les programmes de l'industrie. D'autre part les agents d'activation utilisés dans ce brevet ne sont pas utilisables sur des fragments peptidiques sans apparition d'une racémisation importante.

L'ensemble de la réaction de la présente invention peut être schématisé par les équations suivantes :

(A) 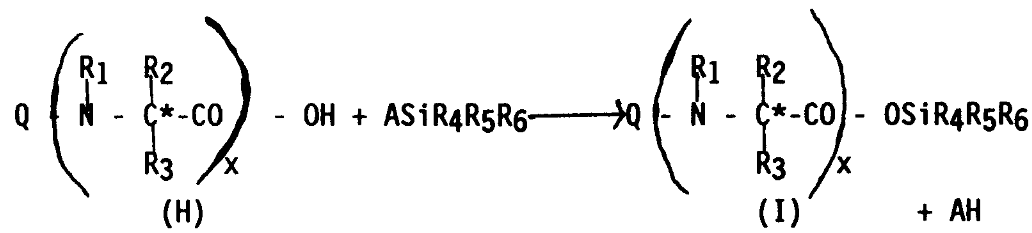

(H) (I)

(B) 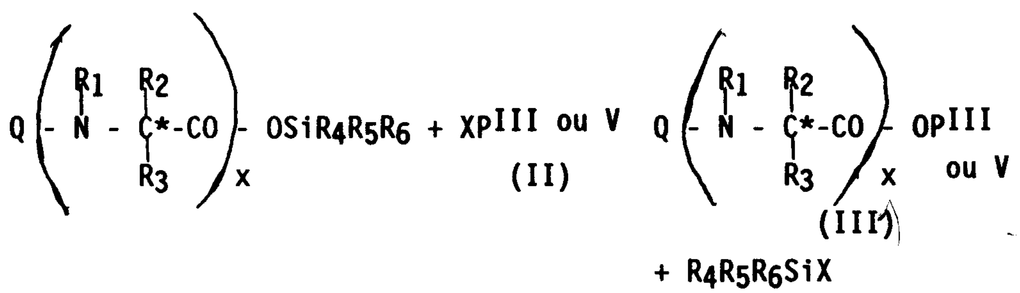

(II) (III)

(C) 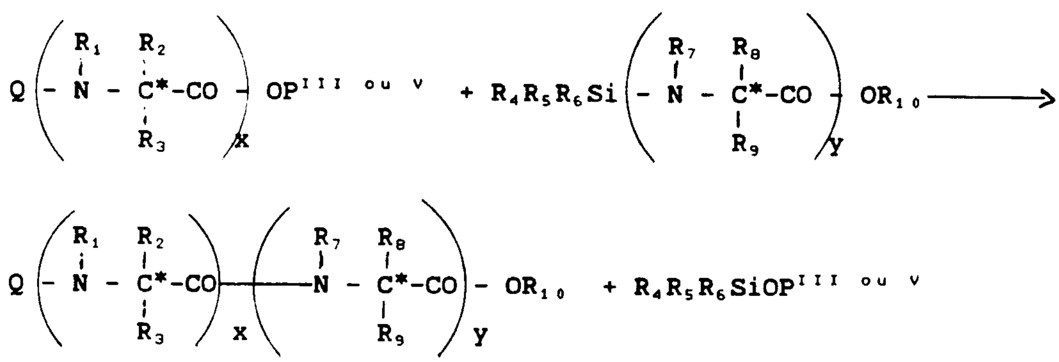

dans lesquelles :

. A représente le chlore ou un groupe

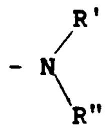

dans lequel :

* R′ représente l'hydrogène, un groupe alkyle $C_nH_{2n+1}$ avec n compris entre 1 et 4,
* R″ représente un groupe alkyle $C_nH_{2n+1}$ avec n compris entre 1 et 4 ou un groupe trialkylsilyl dans lequel le groupe alkyl contient 1 à 4 atomes de carbone,
* R′ et R″ peuvent aussi former un groupe alkylsilyloxyalkylidène et notamment le groupe

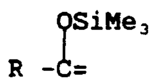

- R pouvant être un méthyl ou un groupe trifluorométhyl.

. Q est un des groupes protecteurs de la fonction amine N-terminale utilisés dans les méthodes connues dans l'art antérieur (Gross et Meienhofer, The peptides, Vol. 3, Academic Press, 1981). A titre d'exemples non limitatifs, Q peut être le groupe t-butyloxycarbonyle (BOC), benzyloxycarbonyle (Z), fluorénylméthyloxycarbonyle (Fmoc), benzoyle, trifluoroacétyle, formyle ;

. $R_1$ et $R_7$ sont soit un hydrogène, soit un groupe méthyle

. $R_2$ et $R_8$ peuvent être choisis parmi les substituants suivants : l'hydrogène, les groupes alkényles ou alkyles $C_nH_{2n+1}$ linéaires ou ramifiés, n possédant des valeurs entières comprises entre 1 et 4, un groupe benzyle ou l'un des groupes dont la liste non limitative figure ci-après :

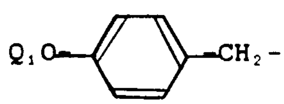 , $Q_2OCH_2-$ , 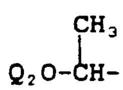 , $Q_3SCH_2-$ , $CH_3S-CH_2-CH_2-$ ,

$Q_8HN-CO-CH_2-$ , $Q_8HN-CO-CH_2-CH_2-$ , $R_{10}O_2C-CH_2-$ , $R_{10}O_2C-CH_2-CH_2-$ ,

$Q_4-NH-(CH_2)_4-$ , 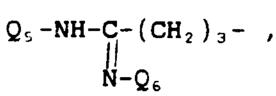 , 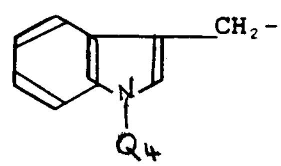 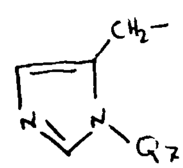

dans lesquels $Q_1$, $Q_2$, $Q_3$, $Q_4$, $Q_5$, $Q_6$, $Q_7$ et $Q_8$ sont des groupes protecteurs des chaînes latérales utilisés dans les méthodes connues dans l'art antérieur (Gross et Meienhofer, The peptides, Vol. 3, Academic Press, 1981). A titre d'exemples non limitatifs, $Q_1$ peut être le groupe benzyle, bromo-2 benzyle ou dichloro-2,6 benzyle, $Q_2$ le groupe benzyle ou t-butyle, $Q_3$ le groupe benzyle, t-butyle, trityle, acétamidométhyle ou benzamidométhyle, $Q_4$ le groupe trifluoroacétyle, t-butyloxycarbonyle ou benzyloxycarbonyle, $Q_5$ peut être le groupe nitro, p-méthoxybenzènesulfonyle ou mésitylènesulfonyle lorsque $Q_6$ = H, ou bien $Q_5$ et $Q_6$ peuvent être simultanément constitués par le groupe adamantyloxycarbonyle, $Q_7$ peut être le groupe phénacyle, benzyloxyméthyle ou t-butoxyméthyle, $Q_8$ peut être enfin le groupe benzhydryle, dimethoxybenzydryle ou xanthydryle.

. $R_3$ et $R_9$ sont choisis parmi les substituants : hydrogène, ou un groupe alkyle $C_nH_{2n+1}$ avec n compris entre 1 et 4.

. $R_1$ avec $R_2$ ou $R_7$ avec $R_8$ peuvent aussi former une chaîne cyclopolyméthylénique contenant 2 à 5 atomes de carbone,

les atomes de carbone sur lesquels sont fixés les substitutions $R_2$ et $R_8$ possèdent la configuration L ou D, ils sont asymétriques (*) sauf si $R_2 = R_3$ et $R_8 = R_9$

. x et y sont des nombres entiers compris entre 1 et 15

. $R_4$, $R_5$ et $R_6$ sont choisis parmi l'hydrogène, les groupes alkyles $C_nH_{2n+1}$, avec n compris entre 1 et 4, à condition que ces trois termes ne soient pas simultanément des hydrogènes,

. $R_{10}$ est un des groupes protecteurs de la fonction acide C-terminale et des fonctions acides des chaînes latérales utilisés dans les méthodes connues dans l'art antérieur (Gross et Meienhofer, The peptides, Vol. 3, Academic Press, 1981). A titre d'exemples non limitatifs, on peut citer les groupes méthyle, éthyle, phényle benzyle ou t-butyle.

Le réactif phosphoré $XP^{III \text{ ou } V}$ est choisi parmi les dérivés du phosphore à l'état d'oxydation III ou V et notamment parmi

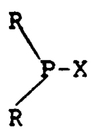 , $(RZ)_2P-X$ , 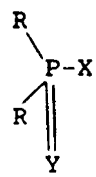 , 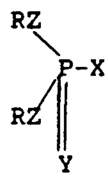 , 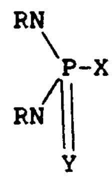 , 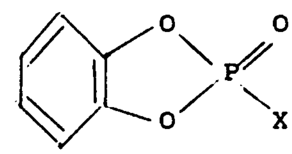

IV V

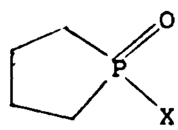 , 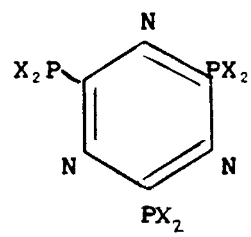

dans lesquels :

X est un chlore ou un brome

R est un groupe alkyle $C_nH_{2n+1}$, avec n compris entre 1 et 4, un groupe phényle, ou bien RN représente un groupe oxo-2 oxazolidinyle-3

Z et Y sont des atomes d'oxygène ou de soufre.

Plus particulièrement, on utilisera le chlorure de diphénylphosphinyle (Dpp-Cl) (formule IV dans laquelle $R = C_6H_5$, $Y = 0$, $X = Cl$) et le chlorure de N, N'-bis-oxo-2 oxazolidinyl-3 phosphinyle (BOP-Cl) (formule V dans laquelle RN =

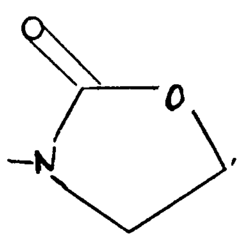

Y = O, X = Cl).

Dans ces conditions, les dérivés activés (III) sont des anhydrides mixtes de formule générale.

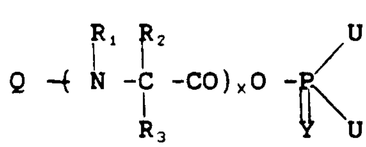

avec U = R, -ZR ou -NR, R, Z et Y ayant les valeurs précédemment définies.

La réaction de silylation de la fonction acide est de préférence réalisée à partir d'un aminoacide ou d'un peptide N-protégé, par réaction avec un réactif silylant de formule $ASiR_4R_5R_6$ dans laquelle les termes A,$R_4$,$R_5$ et $R_6$ ont la même signification que précédemment.

La réaction de silylation de la fonction amine est de préférence réalisée à partir d'un aminoacide ou d'un peptide par réaction entre un ester correspondant et un réactif silylant de formule $ASiR_4R_5R_6$ dans laquelle les termes A, $R_4$, $R_5$ et $R_6$ ont la même signification que précédemment.

Ces réactions de silylation sont effectuées dans les conditions connues dans l'art antérieur.

Les dérivés de l'aminoacide ou du peptide sont introduits avec l'agent silylant dans un solvant tel que par exemple un éther (tétrahydrofuranne), un solvant aliphatique halogéné, un ester, un nitrile (acétonitrile) ou un amide (DMF).

Selon une méthode de mise en oeuvre on utilise notamment des concentrations en aminoacide ou peptide dans le solvant comprise entre 0,1 et 1 mole par litre.

On utilise avantageusement des concentrations en agent silylant par rapport à l'aminoacide ou au peptide comprises entre 1 et 3 moles.

Pour une meilleure mise en oeuvre de l'invention il est préférable au cours de la deuxième étape qui consiste à mettre en contact un peptide ou un amino-acide dont la fonction amine est protégée et la fonction acide est O-silylé avec un dérivé organique du phosphore, d'opérer selon une 1ère méthode sous un courant de gaz inerte tel que l'azote ou l'argon, dans un solvant aliphatique ou aromatique.

On peut utiliser tout solvant permettant de solubiliser le peptide ou l'aminoacide activé et ne réagissant ni avec lui ni avec le dérivé phosphoré. Le solvant est choisi notamment parmi le chlorure de méthylène, le chloroforme, le dichloro-1,2 éthane, le chlorobenzène, l'acétonitrile, le tétrahydrofuranne.

Selon une deuxième méthode de mise en oeuvre du procédé selon l'invention il est encore possible lors de l'activation du peptide ou de l'aminoacide par le dérivé phosphoré d'ajouter dans la solution des époxydes choisis parmi les époxy-1,2 alcanes ou cycloalcanes. On préfère utiliser l'époxy-1,2 cyclopentane et l'époxy-1,2 propane. On préfère dans cette deuxième méthode de mise en oeuvre de l'invention utiliser comme solvant le chloroforme ou le chlorure de méthylène. La température de réaction peut varier notamment de -10 ° C à 30 ° C.

On utilise de préférence une quantité de réactif phosphoré telle que le rapport molaire dudit réactif à l'aminoacide ou au peptide soit compris entre 0,8 et 0,95.

La réaction entre le dérivé phosphoré et l'aminoacide ou le peptide est réalisée de préférence à une température comprise entre - 15 et 30 ° C. On utilise de préférence une concentration molaire du dérivé phosphoré ou de l'aminoacide ou du peptide dans le solvant compris entre 0,01 et 0,1 mole/litre de solvant.

La durée de la réaction d'activation varie avantageusement entre 30 minutes et 2 heures selon le 1er procédé de mise en oeuvre de la 2ème étape de l'invention et entre 10 minutes et 1 heure selon le 2ème procédé de mise en oeuvre de la 2ème étape de l'invention.

En ce qui concerne la réaction de couplage (C) il est connu dans l'art antérieur tel que dans le brevet européen n ° 184243 de coupler des dérivés silylés sur la fonction carboxyle et sur la fonction aminée mais dans aucun de ces procédés n'est décrit le couplage entre un dérivé activé par un groupe phosphoré et un dérivé silylé sur la fonction azotée. Or ce couplage bien précis apporte des résultats quant à l'absence de racémisation tout à fait inattendus.

La réaction de couplage ou troisième étape du procédé de l'invention est réalisée par addition de préférence du composé aminé silylé à la solution du peptide ou de l'aminoacide activé sous atmosphère de gaz inerte et de préférence à une température comprise entre - 10 et 20 ° C. On ajoute avantageusement un catalyseur métallique choisi parmi les halogénures de zinc ou de cuivre.

La durée de la réaction de couplage varie avantageusement entre 2 et 24 heures.

Un autre procédé consiste à introduire dans le milieu réactionnel avant l'addition du composé aminé, un additif qui, à titre d'exemple non limitatif, est choisi parmi les N-hydroxysuccinimide, le N-hydroxy norbornène-5 dicarboximide-2,3, l'hydroxy-1 benzotriazole, la dihydro-3,4 hydroxy-3 céto-4 benzotriazine-1,2,3. Cet additif peut être utilisé sous forme soit de sel de césium, soit de sel de tétraalkylammonium, soit de sel de tétraalkylphosphonium.

L'invention sera plus complètement décrite à l'aide des exemples suivants qui ne doivent en aucun cas être considérés comme limitatifs de l'invention.

Exemple 1

0,43 g (1,94 mM) de benzoyl-L-valine et 0,5 g (3,8 mM) de diméthylsilyldiéthylamine dans 20 ml de chlorure de méthylène anhydre (stabilisé par de l'amylène) sont agités sous azote 5 h à la température ambiante. On concentre alors sous vide sans chauffer. Le résidu brut d'ester diméthylsilylique de la

benzoyl-L-valine est repris par 70 ml de chlorure de méthylène. On ajoute 0,46 g (1,81 mM) de BOP-Cl, qui reste en suspension. On applique durant 2 min un léger vide, puis fait barboter dans le milieu réactionnel un fort courant d'azote pendant 45 min. Le chlorure de diméthylsilyle est entraîné avec une partie du solvant, ce qui provoque une chute de température jusqu'à -15° C. On ajuste le volume final à 35 ml par addition d'une quantité supplémentaire de solvant (de façon à être en solution 0,05 M). Le réactif d'activation est alors complètement dissous. On ajoute 0,25 g (1,81 mM) de chlorure de zinc anhydre et fait barboter de l'azote pendant encore 2 min. On ajoute enfin 0,36 g (1,81 mM) d'ester méthylique de la N-triméthylsilyl-L-valine, 0,304 g (3,62 mM) d'époxy-1,2 cyclopentane, 73 $\mu$l (1,81 mM) de méthanol et agite 16 h à température ambiante sous azote.

On ajoute alors 0,53 g (1,81 mM) du dipeptide Bz-Val-Gly-OMe, qui servira de test pour la détermination du rendement en chromatographie liquide haute performance. On lave 3 fois avec une solution normale d'acide citrique, puis 3 fois avec une solution saturée de bicarbonate de sodium, et enfin une fois à l'eau. La phase organique est séchée sur sulfate de magnésium et le solvant chassé sous vide.

Le rendement en Bz-Val-Val-OMe et le taux de racémisation (exprimé en % DL) sont déterminés à partir du résidu brut par chromatographie liquide haute performance en phase inverse, sur colonne C 18 Ultrasphere Altex, avec comme éluant un mélange MeOH 52 %-$H_2O$ 48 % et un débit de 1 ml/min (détection à 254 nm).
Rendement 64 % DL % 0

Exemple 2

On opère comme dans l'exemple 1, mais avec un temps de couplage de 24 h.
Rendement 64 % DL % 1.

Exemple 3

On opère comme dans l'exemple 1, mais en solution 0,2 M.
Rendement 68 % DL % 2,5.

Exemple 4

On opèra comme dans l'exemple 1, mais sans addition de chlorure de zinc.
Rendement 50 % DL % 11,7.

Exemple 5

0,43 g (1,94 mM) de benzoyl-L-valine et 0,5 g (3,8 mM) de diméthylsilyldiéthylamine dans 20 ml de chlorure de méthylène sont agités sous azote 5 h à la température ambiante. On concentre ensuite sous vide sans chauffer. Le résidu brut est repris par 70 ml de $CH_2Cl_2$. On ajoute 0,46 g (1,81 mM) de BOP-Cl, applique durant 2 min un léger vide, puis fait barboter un fort courant d'azote durant 45 minutes. Le chlorure de diméthylsilyle est entraîné avec une partie du solvant, ce qui entraîne une chute de température jusqu'à - 15° C. On ajuste le volume final à 35 ml (solution 0,05 M) par addition de solvant. On ajoute alors 0,25 g (1,81 mM) de chlorure de zinc anhydre et fait barboter l'azote pendant encore 2 min. On ajoute enfin 0,36 g (1,81 mM) d'ester méthylique de la N-triméthylsilyl L-proline, 0,304 g (3,62 mM) d'époxy-1,2 cyclopentane,73 $10^{-3}$ ml (1,81 mM) de méthanol et agite 16 h sous azote.

On ajoute alors 0,53 g (1,81 mM) du dipeptide test Bz-Val-Gly-OMe, lave 3 fois avec une solution N d'acide citrique, 3 fois avec du bicarbonate de sodium saturé et une fois à l'eau. On sèche sur sulfate de magnésium et chasse le solvant sous vide.

Le rendement en Bz-Val-Pro-OMe et le taux de racémisation (exprimé en DL %) sont déterminés à partir du résidu brut par chromatographie liquide haute performance en phase inverse, sur colonne C 18 Ultrasphere Altex, avec comme éluant un mélange MeOH 48 % - $H_2O$ 52 % et un débit de 1 ml/min (détection à 254 nm).

Rendement 68 % DL 0,2 %

Exemple 6

0,43 g de benzoyl-L-valine (1,94 mM) et 1 ml d'hexaméthyldisilazane dans 20 ml de chlorure de méthylène sont agités sous azote 2 h à la température ambiante. On concentre alors sous vide sans

chauffer. Le résidu brut d'ester triméthylsilylique de la benzoyl-L-valine est repris par 35 ml de chlorure de méthylène. On ajoute successivement 0,304 g (3,62 mM) d'époxy-1,2 cyclopentane et 0,46 g (1,81 mM) de BOP-Cl. Après 20 min d'agitation à la température ambiante sous azote, on introduit dans la solution 0,25 g (1,81 mM) de chlorure de zinc anhydre, puis après 5 min d'agitation 0.37 g (1,81 mm) d'ester méthylique de la N-triméthylsilyl-L-valine et 73 $10^{-3}$ ml (1,81 mM) de méthanol. La réaction est agitée sous azote 16 h à la température ambiante.

Le traitement et l'analyse par chromatographie liquide haute performance sont effectués comme dans l'exemple 1 :

Rendement 74 % DL % 0

Exemple 6 comparatif

A 40 mg (1,81 mM) de benzoyl-L-valine dissous dans 20 ml de chlorure de méthylène anhydre, on ajoute successivement 0,46 mg (1,81 mM) de BOP-Cl et 0,25 ml (1,81 mM) de triéthylamine à - 30 °C. Après 15 minutes d'agitation à cette température, ou ajoute 0,37 g (1,81 mM) de l'ester méthylique de la benzoyl-L-valine et 0,25 ml (1,81 mM) de triéthylamine. On laisse revenir à la température ambiante et poursuit l'agitation durant 15 heures. On lave alors 3 fois à l'acide citrique N, 3 fois avec une solution saturée de bicarbonate de sodium, et finalement à l'eau. La phase organique est séchée sur sulfate de magnésium et le solvant est chassé sous vide.

Rendement 60% Racémisation totale.

Exemple 7

0,63 g (1,94 mM) de l'ester β-benzylique de l'acide N-t-butyloxycarbonyl L-aspartique et un excès d'hexaméthyldisilazane dans 20 ml de $CH_2Cl_2$ sont agités sous azote 2 h à la température ambiante. On concentre sous vide sans chauffer et reprend le résidu par 36 ml de chlorure de méthylène. On ajoute alors 0,304 g (3,62 mM) d'époxy-1,2 cyclopentane, puis 0,46 g (1,81 mM) de BOP-Cl. Après 20 min d'agitation à la température ambiante sous azote, on introduit 0,25 g (1,81 mM) de chlorure de zinc. Après 5 min d'agitation à la température ambiante, on ajoute 0,46 g (1,81 mM) d'ester méthylique de la N-triméthylsilyl L-phénylalanine, 73 $10^{-3}$ ml (1,81 mM) de méthanol, et agite 16 h à la température ambiante sous azote.

On effectue successivement 3 lavages à l'acide citrique N, 3 lavages avec une solution saturée de bicarbonate de sodium et un lavage à l'eau, sèche sur sulfate de magnésium et chasse le solvant sous vide.

Le rendement en Boc-Asp(OBzl)-Phe-OMe est de 50 %. Huile caractérisée par son spectre RMN et par son spectre de masse.

La déprotection en Aspartame est finalement réalisée selon les méthodes de l'art.

Exemple 8

0,43 g de benzoyl-L-valine (1,94 mM) et 1 ml d'hexaméthyldisilazane dans 20 ml de chlorure de méthylène sont agités sous azote 2 h à la température ambiante. On concentre alors sous vide sans chauffer. Le résidu brut d'ester triméthylsilylique de la benzoyl-L-valine est repris par 35 ml de chlorure de méthylène. On ajoute successivement 0,304 g (3,62 mM) d'époxy-1,2 cyclopentane et 0,43 g (1,81 mM) de Dpp-Cl commercial contenant 2 % d'acide diphénylphosphinique. Après 20 min d'agitation à la température ambiante sous azote, on introduit dans la solution 0,41 g (1,81 mM) de bromure cuivrique sec, puis après 5 min d'agitation 0,36 g (1,81 mM) d'ester méthylique de la N-triméthylsilyl L-proline, et 0,304 g (3,62 mM) d'époxy-1,2 cyclopentane. On agite finalement 16 h sous azote à la température ambiante.

Rendement 70 % DL 1,3 %.

Exemple 9

On opère comme dans l'exemple 8, mais en remplaçant le bromure cuivrique par du bromure zincique (1,81 mM)

Rendement 67 % DL 0,7 %

Exemple 10

On opère comme dans l'exemple 8, mais en utilisant de l'iodure cuivrique sec (1,81 mM)
Rendement 82 % DL 0,9 %

Exemple 11

A une solution de 3 g (18,4 mM) de dihydro-3,4 hydroxy-3 céto-4 benzotriazine-1,2,3 dans 20 ml d'eau distillée et 20 ml d'éthanol, on ajoute progressivement une solution aqueuse de 3 g (9,2 mM) de carbonate de césium. On concentre ensuite sous vide jusqu'à sec et le résidu jaune est séché par entraînement azéotropique. Rendement 90 %.

On opère comme dans l'exemple 1, à partir de 0,43 g (1,94 mM) de benzoyl-L-valine et de 0,43 g de DppCl (1,81 mM), mais en remplaçant l'addition de chlorure de zinc par celle de 0,53 g (1,81 mM) de sel de césium de la dihydro-3,4 hydroxy-3 céto-4 benzotriazine-1,2,3. On laisse agiter sous azote 15 minutes en laissant la température remonter jusqu'à 0°. On ajoute alors 0,36 g (1,81 mM) d'ester méthylique de la N-triméthylsilyl-L-valine et agite 2 heures à la température ambiante sous azote.

Rendement quantitatif DL 0,2 %

**Revendications**

1. Procédé de préparation de synthons peptidiques optiquement purs caractérisé en ce que dans une première étape on prépare un dérivé O-Silylé d'un peptide ou d'un aminoacide dont la fonction azotée est protégée,
dans une deuxième étape on active le peptide ou l'aminoacide O-Silylé par un dérivé phosphoré, (X, $P^{III \text{ ou } V}$ avec X chlore ou brome),
dans une troisième étape on condense le peptide ou l'aminoacide activé sur un peptide ou un aminoacide dont la fonction amine a été N sylilée.

2. Procédé selon la revendication 1 caractérisé en ce que le synthon peptidique activé par le dérivé du phosphore contient plus de 1 aminoacide.

3. Procédé selon la revendication 1 caractérisé en ce que on opère au cours des trois étapes en l'absence de dérivés basiques.

4. Procédé selon la revendication 1 caractérisé en ce que le dérivé phosphoré est choisi parmi les dérivés suivants :

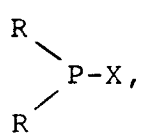
$(RZ)_2P-X$,
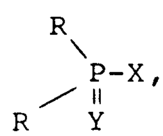
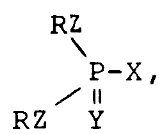
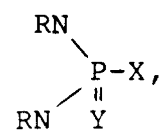

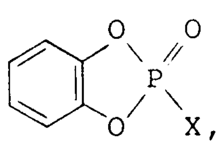
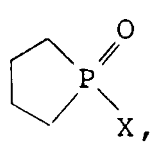
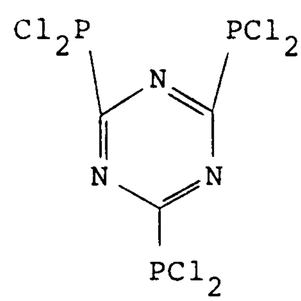

dans lesquels
X représente le chlore ou le brome,
R représente un groupe alkyle $C_nH_{2n+1}$, avec n compris entre 1 et 4, un groupe phényle, ou RN

représente un groupe oxo-2 oxazolidinyle-3.
Z et Y sont des atomes d'oxygène ou de soufre.

5. Procédé selon la revendication 4 caractérisé en ce que le dérivé phosphoré est choisi parmi le chlorure de diphénylphosphinyle (Dpp-Cl) et le chlorure de N, N′-bis oxo-2 oxazolidinyl-3 phosphinyle (BOPCl).

6. Procédé selon la revendication 1 caractérisé en ce que la deuxième étape est réalisée sous courant de gaz inerte, dans un solvant aliphatique ou aromatique.

7. Procédé selon la revendication 6 caractérisé en ce que le solvant est choisi parmi le chlorure de méthylène, le chloroforme, le dichloro-1,2 éthane, le chlorobenzène, l'acétonitrile, le tétrahydrofuranne.

8. Procédé selon les revendications 1 et 6 à 7 caractérisé en ce que la deuxième étape est réalisée de préférence à une température comprise entre - 15 et 30 °C.

9. Procédé selon la revendication 1 caractérisé en ce que lors de la deuxième étape on utilise un rapport molaire du dérivé phosphoré à l'aminoacide ou au peptide compris entre 0,8 et 0,95.

10. Procédé selon la revendication 1 caractérisé en ce que lors de la deuxième étape on utilise un rapport molaire de l'aminoacide ou du peptide au solvant compris entre 0,01 et 0,1 mole/litre.

11. Procédé selon la revendication 1 caractérisé en ce que la deuxième étape est réalisée en présence d'époxydes choisis parmi les époxy-1,2 alcanes ou cycloalcanes.

12. Procédé selon la revendication 11 caractérisé en ce que le solvant choisi est le chloroforme ou le chlorure de méthylène.

13. Procédé selon la revendication 1 caractérisé en ce que au cours de la troisième étape on ajoute avantageusement un catalyseur choisi parmi les halogénures de zinc ou de cuivre.

14. Procédé selon la revendication 1 caractérisé en ce que au cours de la troisième étape on ajoute avantageusement un sel de césium, un sel de tétraalkylammonium ou un sel de tétraalkylphosphonium d'un additif choisi parmi de N-hydroxysuccinimide, le N-hydroxynorbornène-5 dicarboximide-2,3 l'hydroxy-1 benzotriazole ou le dihydro-3,4, hydroxy-3 céto-4 benzotriazine-1,2,3.

## Claims

1. Process for preparing optically pure peptide synthons, characterized in that, in a first stage, an O-silyl derivative of a peptide or amino acid, in which the nitrogenous group is protected, is prepared,
in a second stage, the O-silyl peptide or amino acid is activated by a phosphorus derivative, (X, $P^{III \text{ or } V}$ with X being chlorine or bromine),
in a third stage, the activated peptide or amino acid is condensed with a peptide or amino acid in which the amine group has been N-silylated.

2. Process according to Claim 1, characterised in that the peptide synthon activated by the phosphorus derivative contains more than 1 amino acid.

3. Process according to Claim 1, characterised in that the three stages are performed in the absence of basic derivatives.

4. Process according to Claim 1, characterised in that the phosphorus derivative is chosen from the following derivatives:

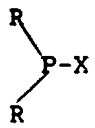 , $(RZ)_2P-X$ , 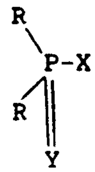 , 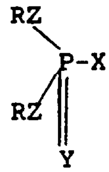 , 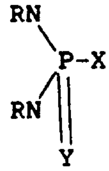 ,

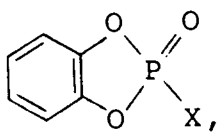 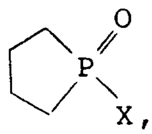 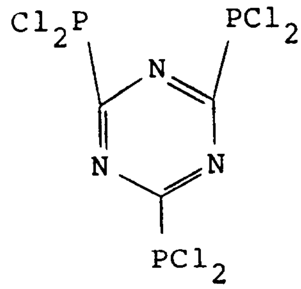

in which
X denotes chlorine or bromine,
R denotes an alkyl group $C_nH_{2n+1}$, with n between 1 and 4, or a phenyl group, or RN denotes a 2-oxo-3-oxazolidinyl group,
Z and Y are oxygen or sulphur atoms.

**5.** Process according to Claim 4, characterised in that the phosphorus derivative is chosen from diphenylphosphinyl chloride (Dpp-Cl) and N,N'-bis(2-oxo-3-oxazolidinyl)phosphinyl chloride (BOPCl).

**6.** Process according to Claim 1, characterised in that the second stage is carried out under a stream of inert gas, in an aliphatic or aromatic solvent.

**7.** Process according to Claim 6, characterised in that the solvent is chosen from methylene chloride, chloroform, 1,2-dichloroethane, chlorobenzene, acetonitrile and tetrahydrofuran.

**8.** Process according to Claims 1 and 6 to 7, characterised in that the second stage is preferably carried out at a temperature of between -15 and 30 °C.

**9.** Process according to Claim 1, characterised in that, during the second stage, a mole ratio of the phosphorus derivative to the amino acid or peptide of between 0.8 and 0.95 is used.

**10.** Process according to Claim 1, characterised in that, during the second stage, a mole ratio of the amino acid or peptide to the solvent of between 0.01 and 0.1 mole/litre is used.

**11.** Process according to Claim 1, characterised in that the second stage is carried out in the presence of epoxides chosen from 1,2-epoxyalkanes or cycloalkanes.

**12.** Process according to Claim 11, characterised in that the chosen solvent is chloroform or methylene chloride.

**13.** Process according to Claim 1, characterised in that, during the third stage, a catalyst chosen from zinc halides or copper halides is advantageously added.

**14.** Process according to Claim 1, characterised in that, during the third stage, a caesium salt, a tetraalkylammonium salt or a tetraalkylphosphonium salt of an additive chosen from N-hydroxysuccinimide, N-hydroxy-5-norbornene-2,3-dicarboximide, 1-hydroxybenzotriazole or 3,4-dihydro-3-hydroxy-4-keto-1,2,3-benzotriazine is advantageously added.

## Patentansprüche

1. Verfahren zur Herstellung optisch reiner Peptid-Synthons dadurch gekennzeichnet, daß man in einer ersten Stufe ein O-silyliertes Derivat eines Peptids oder einer Aminosäure herstellt, deren Stickstofffunktion geschützt ist,
man in einer zweiten Stufe das O-silylierte Peptid oder die O-silylierte Aminosäure durch ein Phosphorderivat aktiviert (X, $P^{III \text{ oder } V}$ mit X gleich Chlor oder Brom),
man in einer dritten Stufe das aktivierte Peptid oder die aktivierte Aminosäure an ein Peptid oder eine Aminosäure kondensiert, deren Aminofunktion N-silyliert wurde.

2. Verfahren nach Anspruch 1, dadurch gekennzeichnet, daß das durch das Phosphorderivat aktivierte Peptid-Synthon mehr als eine Aminosäure enthält.

3. Verfahren nach Anspruch 1, dadurch gekennzeichnet, daß man im Lauf der dreistufigen Synthese in Abwesenheit organischer Basen arbeitet.

4. Verfahren nach Anspruch 1, dadurch gekennzeichnet, daß das Phosphorderivat aus den folgenden Derivaten ausgewählt wird:

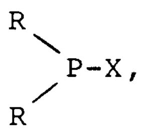 $(RZ)_2P$-X, 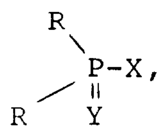 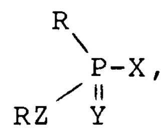 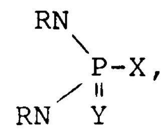

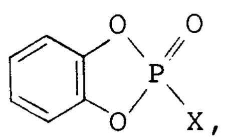 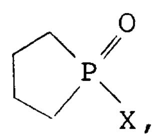 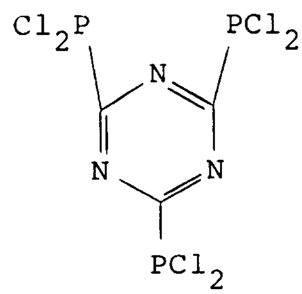

bei denen
X für Chlor oder Brom steht,
R für eine Alkylgruppe $C_nH_{2n+1}$ mit n zwischen eins und vier steht, eine Phenylgruppe, oder RN für eine 2-oxo-3-oxazolidinylgruppe.
Z und Y sind Sauerstoff- oder Schwefelatome.

5. Verfahren nach Anspruch 4, dadurch gekennzeichnet, daß das Phosphorderivat unter dem Diphenylphosphinylchlorid (Dpp-Cl) und dem N,N'-Bis-2-oxo-3-oxazolidinylphosphinyl (BOPCl) ausgewählt wird.

6. Verfahren nach Anspruch 1, dadurch gekennzeichnet, daß die zweite Stufe unter Inertgasfluß durchgeführt wird, in einem aliphatischen oder aromatischen Lösungsmittel.

7. Verfahren nach Anspruch 6, dadurch gekennzeichnet, daß das Lösungsmittel gewählt wird unter Methylenchlorid, Chloroform, 1,2-Dichlorethan, Chlorbenzol, Acetonitril, Tetrahydrofuran.

8. Verfahren nach den Ansprüchen 1 und 6 bis 7, dadurch gekennzeichnet, daß die zweite Stufe bevorzugt bei einer Temperatur zwischen -15 und 30 °C abläuft.

**9.** Verfahren nach Anspruch 1, dadurch gekennzeichnet, daß man bei der zweiten Stufe mit einem molaren Verhältnis des Phosphorderivats zur Aminosäure oder zum Peptid arbeitet, das zwischen 0,8 und 0,95 liegt.

**10.** Verfahren nach Anspruch 1, dadurch gekennzeichnet, daß man bei der zweiten Stufe ein molares Verhältnis der Aminosäure oder des Peptids zum Lösungsmittel benutzt, das zwischen 0,01 und 0,1 Mol/Liter liegt.

**11.** Verfahren nach Anspruch 1, dadurch gekennzeichnet, daß bei der zweiten Stufe in Gegenwart von Epoxiden gearbeitet wird, die unter den 1,2-Epoxyalkanen oder -Cycloalkanen ausgewählt werden.

**12.** Verfahren nach Anspruch 11, dadurch gekennzeichnet, daß das gewählte Lösungsmittel Chloroform oder Methylenchlorid ist.

**13.** Verfahren nach Anspruch 1, dadurch gekennzeichnet, daß man bei der dritten Stufe bevorzugt einen Katalysator hinzufügt, der unter den Zink- oder Kupferhalogeniden ausgewählt wird.

**14.** Verfahren nach Anspruch 1, dadurch gekennzeichnet, daß man bei der dritten Stufe bevorzugt ein Cäsiumsalz hinzufügt, ein Tetraalkylammoniumsalz oder ein Tetraalkylphosphoniumsalz eines Zusatzes ausgewählt unter N-Hydroxysuccinimid, N-5-Hydroxynorbornen-2,3-dicarboximid, 1-Hydroxybenzoltriazol oder 3,4-Dihydro-3-hydroxy-4-keto-1,2,3-benzotriazin.